# EUROPEAN PATENT APPLICATION

(11) **EP 3 991 739 A1**
(43) Date of publication of application: **04.05.2022**
(21) Application number: 19935091.9
(22) Date of filing: 23.07.2019
(51) Int. Cl.: A61K 35/747, A61P 1/00, A61P 29/00, A23L 33/135

(54) **NOVEL PROBIOTIC COMPOSITION FOR REGULATION OF INTESTINAL IMMUNITY**

(30) Priority: 27.06.2019 KR 20190077422
(71) Applicant: Il Dong Pharmaceutical Co., Ltd., Seoul 06752 (KR)
(72) Inventor: MOON, Jin Seok, Seoul 06752 (KR); KIM, Tae-Yoon, Seoul 06752 (KR); KWON, Hyuk-Sang, Seoul 06752 (KR)
(74) Representative: Richardt Patentanwälte PartG mbB
(86) International application number: PCT/KR2019/009117
(87) International publication number: WO 2020/262755

(57) **Abstract**

The present invention relates to a novel probiotic composition for regulation of intestinal immunity and, more specifically, to a composition including *Lactobacillus johnsonii, Lactobacillus plantarum,*
and *Bifidobacterium animalis* subspecies *lactis* as active ingredients for suppressing inflammation and/or preventing, ameliorating, or treating inflammatory bowel diseases.

## Description

### THECHNICAL FIELD

This application claims priority to Korean Patent Application No. 10-2019-0077422 filed on June 27, 2019, and the entire specifications of which are incorporated herein by reference in their entireties.

The present invention relates to a novel probiotic composition for regulation of intestinal immunity, and more particularly, to a composition for preventing, improving, or treating inflammatory bowel disease comprising *Lactobacillus johnsonii, Lactobacillus plantarum* and *Bifidobacterium animalis* subspecies *lactis* as an active ingredient.

### BACKGROUND OF THE INVENTION

Inflammatory bowel disease (IBD) is a disease that causes chronic inflammation in the gastrointestinal tract, and it starts from a relatively young age and is accompanied by symptoms such as abdominal pain, fever, diarrhea, and bleeding. Generally, it is classified into two types: ulcerative colitis (UC) and Crohn's disease (CD). Ulcerative colitis is a type of diffuse nonspecific inflammation of unknown cause of the colon that mainly invades the mucous membrane and frequently forms pimples or ulcers, and is accompanied by various systemic symptoms, including bloody diarrhea. Crohn's disease is a granulomatous inflammatory lesion of unknown cause in which ulcers, fibrosis, stenosis and lesions progress from the mucosa to the entire intestinal tract with discontinuity in the entire digestive tract from the oral cavity to the anus, and is accompanied by systemic symptoms such as abdominal pain, chronic diarrhea, fever, and malnutrition.

The cause of the inflammatory bowel disease has not yet been specifically identified, but it is known that abnormalities in immune function are involved. As immunological factors involved in this, innate immunity, generation of cytokines, activation of CD4, and the like are known. In particular, cytokines are known to play an important role, and it was confirmed that the production of tumor necrosis factor (Tumor nerosis cytokine; TNF-α), interleukin (IL)-1, IL-6, and IL-8 at the site of inflammation was significantly increased in patients with ulcerative colitis and Crohn's disease.

TNF-α, a major proinflammatory cytokine, exacerbates the inflammatory response by increasing the expression of proinflammatory cytokines via the nuclear factor kappa-B pathway. Anti-TNF-a antibodies such as infliximab and adalimumab have been approved for the treatment of patients with severe colitis because inhibition of TNF-α is quite effective in regulating intestinal immunity by preventing apoptosis of mucosal T lymphocytes (non- Patent literature 1). IL-1β recruits granulocytes, causes colon inflammation, and activates CD4+ Th17 cells to secrete IL-17 (Non-Patent literature 2). Neutrophil infiltration is known to be fundamentally necessary for the progression of chronic inflammation. IL-6 promotes neutrophil migration to the colitis region (Non-Patent literature 3).

As a drug used to treat inflammatory bowel disease, steroid immunosuppressants and 5-aminosalicylic acid (5-ASA) drugs that block the production of prostaglandins (e.g., sulfasalazine, etc.), mesalazine, and the like have been used. Although anti-inflammatory drugs and immunosuppressants are prescribed to relieve colitis as exemplified above, it is well known that there are undesirable side effects such as nausea, vomiting, headache, hemolytic anemia and male infertility.

Accordingly, there is a continuous demand for the development of safer and more effective treatments for inflammatory bowel disease.

### [Prior art literature]

(Non- Patent literature 1) Atreya R, Zimmer M, Bartsch B, et al.: Antibodies against tumor necrosis factor (TNF) induce T-cell apoptosis in patients with inflammatory bowel diseases via TNF receptor 2 and intestinal CD14(+) macrophages. Gastroenterology 2011;141:2026-2038.
(Non- Patent literature 2) Coccia M, Harrison OJ, Schiering C, et al.: IL-1beta mediates chronic intestinal inflammation by promoting the accumulation of IL-17A secreting innate lymphoid cells and CD4(+) Th17 cells. J Exp Med 2012;209:1595-1609.
(Non- Patent literature 3) Wang Y, Wang K, Han GC, et al.: Neutrophil infiltration favors colitis-associated tumorigenesis by activating the interleukin-1 (IL-1)/IL-6 axis. Mucosal Immunol 2014;7:1106-1115.

### DETAILED DESCRIPTION OF THE INVENTION

### TECHNICAL PROBLEM

Accordingly, as a result the present inventors have made intensive research efforts to develop a safe and effective therapeutic agent for inflammatory bowel disease, it was confirmed that the prevention and treatment effect of inflammatory bowel disease (IBD) was significantly increased by using the combination of three types of lactic acid bacteria unique to the present invention, in particular, the present invention was completed by confirming that this effect is superior to that of sulfasalazine, which is commonly used as an IBD treatment drug.

Accordingly, an object of the present invention is to provide a pharmaceutical composition for preventing or treating inflammatory bowel disease, comprising *Lactobacillus johnsonii, Lactobacillus plantarum* and *Bifidobacterium animalis* subspecies *lactis* as an active ingredient.

The other object of the present invention is to provide a food composition for preventing or improving inflammatory bowel disease, comprising *Lactobacillus johnsonii, Lactobacillus plantarum,* and *Bifidobacterium animalis* subspecies *lactis* as an active ingredient.

Another object of the present invention is to provide a (pharmaceutical or food) composition for anti-inflammatory, comprising *Lactobacillus johnsonii, Lactobacillus plantarum,* and *Bifidobacterium animalis* subspecies *lactis* as an active ingredient.

Another object of the present invention is to provide a use of a composition comprising *Lactobacillus johnsonii, Lactobacillus plantarum* and *Bifidobacterium animalis* subspecies *lactis* as an active ingredient for preparing a formulation for the prevention or treatment of inflammatory bowel disease.

Another object of the present invention is to provide a method of treating inflammatory intestinal disease, comprising administering to a subject in need thereof an effective amount of a composition comprising *Lactobacillus johnsonii, Lactobacillus plantarum* and *Bifidobacterium animalis* subspecies *lactis* as an active ingredient.

Another object of the present invention is to provide a use of a composition comprising *Lactobacillus johnsonii, Lactobacillus plantarum* and *Bifidobacterium animalis* subspecies *lactis* as an active ingredient for preparing a formulation for preparing an anti-inflammatory agent.

Another object of the present invention is to provide a method of inhibiting inflammation comprising administering to a subject in need thereof an effective amount of a composition comprising *Lactobacillus johnsonii, Lactobacillus plantarum* and *Bifidobacterium animalis* subspecies *lactis* as an active ingredient.

### TECHNICAL SOLUTION

In order to achieve the above object, the present invention provides a pharmaceutical composition for preventing or treating inflammatory bowel disease, comprising *Lactobacillus johnsonii, Lactobacillus plantarum* and *Bifidobacterium animalis* subspecies *lactis* as an active ingredient.

In addition, the present invention provides a pharmaceutical composition for preventing or treating inflammatory bowel disease, consisting of *Lactobacillus johnsonii, Lactobacillus plantarum* and *Bifidobacterium animalis* subspecies *lactis* as an active ingredient.

In addition, the present invention provides a pharmaceutical composition for preventing or treating inflammatory bowel disease, essentially consisting of *Lactobacillus johnsonii, Lactobacillus plantarum* and *Bifidobacterium animalis* subspecies *lactis* as an active ingredient.

In order to achieve another object, the present invention provides a food composition for preventing or improving inflammatory bowel disease, comprising *Lactobacillus johnsonii, Lactobacillus plantarum,* and *Bifidobacterium animalis* subspecies *lactis* as an active ingredient.

In addition, the present invention provides a food composition for preventing or improving inflammatory bowel disease, consisting of *Lactobacillus johnsonii, Lactobacillus plantarum,* and *Bifidobacterium animalis* subspecies *lactis* as an active ingredient.

In addition, the present invention provides a food composition for preventing or improving inflammatory bowel disease, essentially consisting of *Lactobacillus johnsonii, Lactobacillus plantarum,* and *Bifidobacterium animalis* subspecies *lactis* as an active ingredient.

In order to achieve another object, the present invention provides a (pharmaceutical or food) composition for anti-inflammatory, comprising *Lactobacillus johnsonii, Lactobacillus plantarum,* and *Bifidobacterium animalis* subspecies *lactis* as an active ingredient.

In addition, the present invention provides a (pharmaceutical or food) composition for anti-inflammatory, consisting of *Lactobacillus johnsonii, Lactobacillus plantarum,* and *Bifidobacterium animalis* subspecies *lactis* as an active ingredient.

In addition, the present invention provides a (pharmaceutical or food) composition for anti-inflammatory, essentially consisting of *Lactobacillus johnsonii, Lactobacillus plantarum,* and *Bifidobacterium animalis* subspecies *lactis* as an active ingredient.

In order to achieve another object, the present invention provides a use of a composition comprising *Lactobacillus johnsonii, Lactobacillus plantarum* and *Bifidobacterium animalis* subspecies *lactis* as an active ingredient for preparing a formulation for the prevention or treatment of inflammatory bowel disease.

In order to achieve another object, the present invention provides a method of treating inflammatory bowel disease, comprising administering to a subject in need thereof an effective amount of a composition comprising *Lactobacillus johnsonii, Lactobacillus plantarum* and *Bifidobacterium animalis* subspecies *lactis* as an active ingredient.

In order to achieve another object, the present invention provides a use of a composition comprising *Lactobacillus johnsonii, Lactobacillus plantarum* and *Bifidobacterium animalis* subspecies *lactis* as an active ingredient for preparing a formulation for preparing an anti-inflammatory agent.

In order to achieve another object, the present invention provides a method of inhibiting inflammation comprising administering to a subject in need thereof an effective amount of a composition comprising *Lactobacillus johnsonii, Lactobacillus plantarum* and *Bifidobacterium animalis* subspecies *lactis* as an active ingredient.

Hereinafter, the present invention will be described in detail.

In the present invention, the term 'comprising' is used the same as 'including' or 'characterized', and in the composition or method, additional component elements or method steps not mentioned are not excluded. The term 'consisting of' is used the same as 'made of' and means excluding additional elements, steps, or ingredients not specifically described. The term 'essentially consisting of' or 'essentially made of' in the scope of a composition or method, it is meant to include, in addition to the described component elements or steps, the component elements or steps and the like that do not materially affect its basic properties.

Throughout the disclosure herein, various aspects or conditions related to the present invention may be suggested in a range format. In the present specification, the description of the range value includes the corresponding boundary value, that is, includes all values above the lower limit value and below the upper limit value unless otherwise specified. It should be understood that the description in range format is merely for convenience and brevity, and should not be construed as an inflexible limitation on the scope of the present invention. Accordingly, statements of ranges are to be considered as specifically disclosing all possible subranges as well as individual numerical values within that range. For example, a statement of a range such as 7 to 170 refers to individual numbers within the range, such as 9, 27, 35, 101, and 155, as well as subranges such as10 to 127, 23 to 35, 80 to 100, 50 to 169 and the like are to be considered as specifically disclosed. This applies regardless of the width of the range.

In the present invention, the term 'probiotics' is defined as microorganisms that give health benefits to the host when administered in an appropriate amount. Their established safety and beneficial effects on human health have led to the emergence of probiotics as substitutes or complements to medicines. The advantage of probiotics is that they have very few side effects.

The present inventors screened the individual efficacy of each strain in various aspects targeting various lactic acid bacteria strains isolated from infant feces, kimchi, and cheese, in addition, efficacy evaluation was performed in various aspects using various strains in various combinations. As a result, the present inventors specifically confirmed an unexpected synergistic effect in anti-inflammatory efficacy when using a combination of three strains of *Lactobacillus johnsonii* IDCC9203 (KCTC 10923BP), *Lactobacillus plantarum* IDCC3501 (KCTC 13586BP) and *Bifidobacterium animalis* subspecies *lactis* IDCC4301 (KCTC 13587BP), in addition, it was confirmed that substantial therapeutic efficacy in pharmacological terms for inflammatory bowel disease (IBD) is remarkable. In particular, the therapeutic effect of the present invention was superior to that of sulfasalazine, a known IBD treatment drug. Considering that the probiotic characteristics are strain-dependent even in bacteria of the same species, and there is difficulty in preparing a composition that exhibits excellent performance for all probiotic requirements, the three types of lactic acid bacteria composition of the present invention and the remarkable effect confirmed by the present inventors have great technical significance. In addition, compared to the V company's 8 types of lactic acid bacteria mixed formulation, which was previously recognized only for intestinal immunity in Korea, the 3-type strain mixture of the present invention showed excellent therapeutic effect even with a smaller dose.

Accordingly, the present invention provides a pharmaceutical composition for preventing or treating inflammatory bowel disease *Lactobacillus johnsonii, Lactobacillus plantarum,* and *Bifidobacterium animalis* subspecies *lactis* as an active ingredient, in addition, it provides a food composition for preventing or improving inflammatory bowel disease comprising the three strains as an active ingredient.

In addition, the present invention provides a pharmaceutical composition for preventing or treating inflammatory bowel disease, or a food composition for preventing or improving inflammatory bowel disease, consisting of the three strains.

In addition, the present invention provides a pharmaceutical composition for preventing or treating inflammatory bowel disease, or a food composition for preventing or improving inflammatory bowel disease, essentially consisting of the three strains.

Preferably, the present invention provides a probiotic composition comprising (in an effective amount) a *Lactobacillus johnsonii* IDCC9203 strain, *Lactobacillus plantarum* IDCC3501 strain and *Bifidobacterium animalis* subspecies *lactis* IDCC4301 strain, it also provides a probiotic composition consisting of the three strains (in an effective amount), in addition, it provides a probiotic composition essentially consisting of the three strains (in an effective amount).

In one preferred embodiment, the present invention provides a pharmaceutical composition for preventing or treating inflammatory bowel disease comprising a *Lactobacillus johnsonii* IDCC9203 strain, *Lactobacillus plantarum* IDCC3501 strain and *Bifidobacterium animalis* subspecies *lactis* IDCC4301 strain as an active ingredient, in addition, it provides a food composition for preventing or improving inflammatory bowel disease comprising the three strains as an active ingredient.

In another embodiment, the present invention provides a pharmaceutical composition for preventing or treating inflammatory bowel disease consisting of *Lactobacillus johnsonii* IDCC9203 strain, *Lactobacillus plantarum* IDCC3501 strain and *Bifidobacterium animalis* subspecies *lactis* IDCC4301 strain, in addition, it provides a food composition for preventing or improving inflammatory bowel disease consisting of the three strains.

In another embodiment, the present invention provides a pharmaceutical composition for preventing or treating inflammatory bowel disease essentially consisting of *Lactobacillus johnsonii* IDCC9203 strain, *Lactobacillus plantarum* IDCC3501 strain and *Bifidobacterium animalis* subspecies *lactis* IDCC4301 strain, in addition, it provides a food composition for preventing or improving inflammatory bowel disease essentially consisting of the three strains.

If the 'inflammatory bowel disease' is a disease known in the art that an inflammatory condition in the intestine is a major pathology, the type is not particularly limited, but for example, it may be preferably selected from the group consisting of Crohn's disease, ulcerative colitis, intestinal Behcet's disease and ischemic enteritis.

The composition of the present invention is not limited thereto, but may be formulated so that the three types of lactic acid bacteria *(Lactobacillus johnsonii, Lactobacillus plantarum,* and *Bifidobacterium animalis* subspecies *lactis*) are present in one container in the form of a mixture, alternatively, each of the three types of lactic acid bacteria is provided packaged in a separate container, but may be formulated to be administered simultaneously or sequentially at the time of use.

In one embodiment, the three types of lactic acid bacteria of the present invention may be provided in the form of a mixture, as an example of such an embodiment, the present invention provides a pharmaceutical composition for preventing or treating (or food composition for preventing or improving the disease) inflammatory bowel disease comprising a mixed lactic acid bacteria of *Lactobacillus johnsonii, Lactobacillus plantarum,* and *Bifidobacterium animalis* subspecies *lactis* as an active ingredient.

In the composition of the present invention, the effective amount of colony forming units (CFU) of each strain can be determined by a person skilled in the art as desired (disease prevention, health or therapeutic treatment) or necessary, and also can be determined according to the final formulation.

As an example, 1 × 10⁵ to 1 × 10¹⁵ bacteria (number of bacteria or CFU) can be added per 1 g (or ml) of the total composition in the production of food or drug for live or dead cells of each of the three strains of the present invention and may be preferably added in an amount of 1×10⁶ to 1×10¹³, but is not limited thereto.

In addition, the daily intake of the composition containing the above-described live cells or dead cells thereof is 1×10⁵ to 1×10¹⁰ CFU/kg, preferably 1×10⁶ to 1×10⁹ CFU/kg, based on the total number of cells, preferably, it may be 1 × 10⁶ to 1 × 10⁹ CFU / kg as an example, and intake may be ingested once a day, or may be ingested in several divided doses, but is not limited thereto.

In the composition of the present invention, the relative ratio (e.g., combination ratio) in which the three types of lactic acid bacteria are provided is not particularly limited as long as it exhibits the desired effect (anti-inflammatory effect or inflammatory bowel disease prevention/treatment/improvement effect) in the present invention. For example, the composition ratio of *L. johnsonii* IDCC9203: *L. plantarum* IDCC3501: *B. animalis* subspecies *lactis* IDCC4301 is 1-100: 1-100: 1-100 on a cfu/g (or cfu/ml) based on cfu/g unit, preferably it may be formulated in a ratio of 1 to 30: 1 to 30: 1 to 30 based on cfu/g (or cfu/ml) unit, more preferably, in a ratio of 1 to 10: 1 to 10: 1 to 10 based on a cfu/g (or cfu/ml) unit.

In one embodiment, the composition ratio of *L. johnsonii* IDCC9203: *L. plantarum* IDCC3501: B. *animalis* subspecies lactis IDCC4301 is 1: 7: 2 based on a cfu/g (or cfu/ml) unit.

In one preferred embodiment, the composition ratio of the three types of lactic acid bacteria may be one in a ratio of 1:1:1 based on cfu/g (or cfu/ml) unit.

In addition, in the present invention, the three types of lactic acid bacteria may be provided in the form of live cells or dead cells, respectively.

Methods for treating bacteria for providing live preparations in the art are well known in the art, and if the bacteria are in a live form, the form in which they are provided is not particularly limited. For example, after culturing the strains, a cell pellet may be recovered, used as it is, or treated by a desired means, such as by concentration and/or freeze-drying, to be added to the manufacture of pharmaceutical or edible products. Occasionally, probiotic formulations may be subjected to an immobilization or encapsulation process to improve shelf life. Several bacterial immobilization or encapsulation techniques are known in the art.

In the present invention, the term 'dead cells' refers to cells sterilized by heating, pressurization, drug treatment, etc. The production method of the dead cells is not particularly limited as long as it is by the lactic acid bacteria killing method known in the art, and for example, the dead cells of the present invention may be prepared by a killing method including heat treatment (by heat treatment). The heat treatment may be performed only on live cells isolated and obtained from the culture medium, or may be performed on a medium containing the live cells. The heat treatment temperature is not particularly limited as long as the properties of the cells are maintained and other general bacteria are sterilized, it may be carried out at 80 °C to 150 °C, and preferably at 80 °C to 110 °C.

The heat treatment time is not particularly limited as long as a person skilled in the art can obtain the desired preparing quality in consideration of the temperature conditions, for example, it may proceed from 5 minutes to 15 minutes.

During the heat treatment process for producing the dead cells, a person skilled in the art may add any pre-treatment or additional conditions for production efficiency in a line that does not affect the intrinsic functionality of the dead cells of the present invention. For example, distilled water may be added to and mixed with live cells before the heat treatment.

The dead cells may be subjected to any additional process for the preparation of the dead cell preparation (formulation), for example, concentration, drying, etc. may be performed. The drying method is not particularly limited as long as it is a method used for drying lactic acid bacteria in the art, but may be, for example, heat (air) drying or freeze-drying method.

In another embodiment, the present invention provides pharmaceutical composition or food composition for anti-inflammatory, comprising *Lactobacillus johnsonii, Lactobacillus plantarum,* and *Bifidobacterium animalis* subspecies *lactis* as an active ingredient.

Preferably, the present invention provides a pharmaceutical composition or food composition for anti-inflammatory, comprising *Lactobacillus johnsonii* IDCC9203 strain, *Lactobacillus plantarum* IDCC3501 strain and *Bifidobacterium animalis* subspecies *lactis* IDCC4301 strain as an active ingredient.

The pharmaceutical composition of the present invention may include the live or dead cells of the three types of lactic acid bacteria described above alone, or may additionally include one or more pharmaceutically acceptable carriers, excipients or diluents. In the above, 'pharmaceutically acceptable' means a non-toxic composition that is physiologically acceptable and does not inhibit the action of the active ingredient when administered to humans and usually does not cause allergic reactions such as gastrointestinal disorders, dizziness or similar reactions. The pharmaceutically acceptable carrier may further include, for example, a carrier for oral administration or a carrier for parenteral administration.

Carriers for oral administration may include lactose, starch, cellulose derivatives, magnesium stearate, stearic acid, and the like.

The pharmaceutical composition of the present invention may be administered to mammals including humans by any method. For example, it may be administered orally or parenterally (e.g., application method, etc.). In addition, the pharmaceutical composition of the present invention may be formulated as a preparation for oral administration or parenteral administration according to the administration route as described above.

For example, in the case of a formulation for oral administration, the composition of the present invention may be formulated as a freeze-dried powder, capsules, granules, tablets, pills, dragees, liquids, gels, syrups, slurries, suspensions, wafers or the like using a method known in the art. For example, an oral preparation can be provided by mixing the active ingredient with a solid excipient, adding a suitable adjuvant thereto, and then processing it into a capsule or the like. Examples of suitable excipients include sugars including lactose, dextrose, sucrose, sorbitol, mannitol, xylitol, erythritol and maltitol and the like, starches including corn starch, wheat starch, rice starch and potato starch, celluloses including cellulose, methylcellulose, sodium carboxymethylcellulose and hydroxypropylmethyl-cellulose and fillers such as gelatin, polyvinylpyrrolidone, and the like. Furthermore, the pharmaceutical composition of the present invention may further include an anti-aggregating agent, a lubricant, a wetting agent, a flavoring agent, an emulsifier and a preservative, and the like, but is not limited thereto.

The food composition of the present invention includes all foods in a conventional sense, and includes all types of functional foods, nutritional supplements, health food and food additives and the like. Food compositions of this type can be prepared in various forms according to conventional methods known in the art.

The food composition of the present invention may include a health functional food. The term 'health functional food' as used in the present invention refers to food manufactured and processed in the form of tablets, capsules, powders, granules, liquids, and pills using raw materials or ingredients useful for the human body. Here, the term 'functionality' refers to obtaining useful effects for health purposes such as regulating nutrients or physiological effects on the structure and function of the human body. The health functional food of the present invention can be prepared by a method commonly used in the art, and at the time of manufacture, it can be prepared by adding raw materials and components commonly added in the art.

In addition, if the formulation of the health functional food is also recognized as a health functional food, it may be manufactured without limitation. The composition for food of the present invention can be prepared in various forms, and unlike general drugs, it has the advantage that there are no side effects that may occur when taking the drug for a long period of time since it uses food as a raw material, and it is portable and can be taken as a supplement.

For example, as a health functional food, the live bacteria of the three types of lactic acid bacteria or dead cells thereof of the present invention may be encapsulated or granulated and ingested. In addition, the food composition of the present invention may contain various nutrients, vitamins, electrolytes, flavoring agents, colorants, pectic acid and its salts, alginic acid and its salts, organic acids, protective colloidal thickeners, pH regulators, stabilizers, preservatives, and the like. In addition, it may contain the flesh of a fruit. These components may be used independently or in combination. Although the proportion of these additives is not very important, it is generally selected in the range of 0.01 to 0.3 parts by weight per 100 parts by weight of the food composition of the present invention, but is not limited thereto.

In addition, the food composition of the present invention may contain various flavoring agents or natural carbohydrates as additional ingredients. The carbohydrates include monosaccharides such as glucose, fructose, disaccharides such as maltose, sucrose, polysaccharides such as dextrin and cyclodextrin, and sugar alcohols such as xylitol, sorbitol, and erythritol. As the sweetener, natural sweeteners such as taumatine and stevia extract, synthetic sweeteners such as saccharin and aspartame, and the like can be used. The ratio of the natural carbohydrate is generally about 0.01 to 0.04 g, preferably about 0.02 to 0.03 g per 100 mL of the composition of the present invention, but is not limited thereto.

The food composition of the present invention may also be provided in the form of a fermented food. The three types of lactic acid bacteria of the present invention may be contained in various edible products such as dairy products and yogurt, curd, cheese (e.g., quark, cream, processed, soft and hard), fermented milk, milk powder, milk-based fermented products, ice cream, fermented cereal based product, milk formula based powder), beverages, dressings and pet food. As used herein, 'edible product' is used herein in its broadest sense, including all types of products, in any form of presentation, that can be consumed by animals, except for pharmaceutical products and veterinary products. Examples of other edible products include meat products (e.g., liver pastes, sausages, salami sausages or meat spreads) and chocolate spreads, fillings (e.g., truffle, cream) and frosting, chocolate, confectionery (e.g., caramel, fondant or toffee), baked good (cake, pastry), sauces and soups, fruit juice and coffee cream (coffee whitener). Edible products of particular interest are dietary supplements and infant formulas. In an aspect of the present invention, dietary supplements also include nutraceuticals known as food extracts with medicinal effects on human health. Feeds for animal consumption are also included within the scope of the present invention. The composition of the present invention can also be used as a component of other food products.

In addition, the present invention provides a use of a composition comprising *Lactobacillus johnsonii, Lactobacillus plantarum* and *Bifidobacterium animalis* subspecies *lactis* as an active ingredient for preparing a formulation for the prevention or treatment of inflammatory bowel disease.

In addition, the present invention provides the use of a composition consisting of the three strains as an active ingredient for preparing a formulation for the prevention or treatment of inflammatory bowel disease, the present invention provides the use of a composition essentially consisting of the three strains as an active ingredient for preparing a formulation for the prevention or treatment of inflammatory bowel disease.

In addition, the present invention provides a method for treating inflammatory bowel disease, comprising administering to a subject in need thereof an effective amount of a composition comprising *Lactobacillus johnsonii, Lactobacillus plantarum* and *Bifidobacterium animalis* subspecies *lactis* as an active ingredient.

In addition, the present invention provides a use of a composition comprising *Lactobacillus johnsonii, Lactobacillus plantarum* and *Bifidobacterium animalis* subspecies *lactis* as an active ingredient for preparing an anti-inflammatory agent.

In addition, the present invention provides the use of a composition consisting of the three strains as an active ingredient for preparing an anti-inflammatory agent,

It provides the use of a composition essentially consisting of the three strains as an active ingredient for preparing an anti-inflammatory agent.

In addition, the present invention provides a method of inhibiting inflammation comprising administering to a subject in need thereof an effective amount of a composition comprising *Lactobacillus johnsonii, Lactobacillus plantarum* and *Bifidobacterium animalis* subspecies *lactis* as an active ingredient.

'Anti-inflammatory' in the present invention refers to inhibiting molecular and cellular inflammatory reactions such as secretion of pro-inflammatory factors such as infiltration of immune cells, pro-inflammatory cytokines, prostaglandins and nitric oxide (NO), and preventing, alleviating or improving symptoms such as erythema, keratinization, increased skin thickness, redness, fever, and pain.

The 'inflammation inhibition' of the present invention refers to inhibiting molecular and cellular inflammatory responses such as the secretion of pro-inflammatory factors such as infiltration of immune cells, pro-inflammatory cytokines, prostaglandins and nitric oxide (NO) such as the anti-inflammatory described above, and preventing, alleviating or improving symptoms such as erythema, keratinization, increased skin thickness, redness, fever, and pain.

The 'effective amount' of the present invention refers to an amount that, when administered to a subject, exhibits an effect of improving, treating, preventing, detecting, diagnosing, or inhibiting or reducing inflammation and/or inflammatory bowel disease, the 'subject' may be an animal, preferably an animal, including a mammal, particularly a human, and may be an animal-derived cell, tissue, organ, or the like. The subject may be a patient in need of the effect.

The 'treatment' of the present invention refers to ameliorating inflammation and/or inflammatory bowel disease or symptoms of inflammatory and/or inflammatory bowel disease comprehensively, this may include curing, substantially preventing, or ameliorating the condition of inflammation and/or inflammatory bowel disease, and include alleviating, curing or preventing one or most of the symptoms resulting from the disease, but is not limited thereto.

### ADVANTAGEOUS EFFECT

The composition comprising the unique combination of three types of lactic acid bacteria of the present invention has a remarkable preventive and therapeutic effect on inflammatory bowel disease (IBD), and in particular, this effect is pharmacologically superior compared to known IBD therapeutic drugs (e.g., sulfasalazine). In addition, the composition of the present invention has a great advantage as a safe composition with very little risk of side effects due to the nature of the probiotic composition.

### BRIEF DESCRIPTION OF THE DRAWINGS

FIG. 1 shows the results of comparing the inhibitory effect on TNF-α increased in Raw 264.7 cells by LPS treatment by treating *Lactobacillus johnsonii* IDCC9203 (No. 6), *Lactobacillus plantarum* IDCC3501 (No.7) and *Bifidobacterium animalis* subspecies *lactis* IDCC4301 (No.14) individual strain, these lactic acid bacteria three types mixture (represented by Mix), the same type strain mixtures (*Lactobacillus johnsonii* KCTC 3801 ^{T}, *Lactobacillus plantarum* KCTC 3108^{T}, *Bifidobacterium animalis* ssp. *lactis* IDCC KCTC 3219^{T}), respectively (DEX: dexamethasone).
FIG. 2 shows the results of confirming that IL-6 increased in Raw 264.7 cells by the LPS treatment is inhibited by the treatment of the present invention three kinds of lactic acid bacteria preparations.
FIG. 3 shows the results of confirming the degree of change in IL-6 level after administering the type strain mixtures (*Lactobacillus johnsonii* KCTC 3801^{T}, *Lactobacillus plantarum* KCTC 3108^{T}, *Bifidobacterium animalis* ssp. *lactis* IDCC KCTC 3219^{T}) having a homogeneous relationship with the composition of the present invention to LPS-treated Raw 264.7 cells.
FIG. 4 shows the results of confirming that IL-1β increased in Raw 264.7 cells by the LPS treatment is inhibited by the treatment of the three lactic acid bacteria preparations of the present invention.
FIG. 5 shows the results of confirming the degree of change in the IL-1β level after administering the type strain mixtures (*Lactobacillus johnsonii* KCTC 3801^{T}, *Lactobacillus plantarum* KCTC 3108^{T}, *Bifidobacterium animalis* ssp. *lactis* IDCC KCTC 3219^{T}) having a homogeneous relationship with the composition of the present invention to LPS-treated Raw 264.7 cells.
FIG. 6 shows the results of confirming NO (nitric oxide) inhibitory ability by administrating various individual lactic acid strains including *Lactobacillus johnsonii* IDCC9203(No.6), *Lactobacillus plantarum* IDCC3501(No.7) and *Bifidobacterium animalis* subspecies *lactis* IDCC4301 (No.14) strain to LPS-treated Raw 264.7 cells.
FIG. 7 shows the DAI score for each dose administered when the three types of lactic acid bacteria formulations of the present invention were administered to an animal model of colitis, and sulfasalazine, a known IBD treatment, was used as a comparison group.
FIG. 8 is an image of observing the length of the colon when the three types of lactic acid bacteria preparations of the present invention were administered to an animal model of colitis, and sulfasalazine, a known IBD treatment, was used as a comparison group.
FIG. 9 shows the results of measuring the length of the colon when the three types of lactic acid bacteria preparations of the present invention were administered to an animal model of colitis for each dose, and sulfasalazine, a known IBD treatment, was used as a comparison group.
FIG. 10 is an image of a cross-section of the colon when the three types of lactic acid bacteria preparations of the present invention were administered to an animal model of colitis, and sulfasalazine, a known IBD treatment, was used as a comparison group.
FIG. 11 shows comparatively histological scores for colon tissue damage when three types of lactic acid bacteria preparations of the present invention are administered to an animal model of colitis, and sulfasalazine, a known IBD treatment, was used as a comparison group.
FIG. 12 shows the results of measuring the TNF-α level in the colon when the three types of lactic acid bacteria preparations of the present invention were administered to an animal model of colitis for each dose, and sulfasalazine, a known IBD treatment, was used as a comparison group.
FIG. 13 shows the results of measuring the IL-1β level in the colon when the three types of lactic acid bacteria preparations of the present invention were administered to an animal model of colitis for each dose, and sulfasalazine, a known IBD treatment, was used as a comparison group.
FIG. 14 shows the results of measuring the IL-6 level in the colon when the three types of lactic acid bacteria preparations of the present invention were administered to an animal model of colitis for each dose, and sulfasalazine, a known IBD treatment, was used as a comparison group.
FIG. 15 shows the DAI score when the three types of lactic acid bacteria preparations of the present invention or the V company lactic acid bacteria preparations are administered to an animal model of colitis.
FIG. 16 shows an image of observing the length of the colon when the three types of lactic acid bacteria preparations of the present invention or the company V lactic acid bacteria preparations were administered to an animal model of colitis.
FIG. 17 shows the results of measuring the length of the colon when the three types of lactic acid bacteria preparations of the present invention or the V company lactic acid bacteria preparations were administered to an animal model of colitis.
FIG. 18 shows the histological score for colon tissue damage when the three types of lactic acid bacteria preparations of the present invention or the V company lactic acid bacteria preparations are administered to an animal model of colitis.
FIG. 19 shows the results of measuring the level of TNF-α in the colon when the three types of lactic acid bacteria preparations of the present invention or the company V lactic acid bacteria preparations were administered to an animal model of colitis.
FIG. 20 shows the results of measuring the IL-1β level in the colon when the three types of lactic acid bacteria preparations of the present invention or the V company lactic acid bacteria preparations were administered to an animal model of colitis.
FIG. 21 shows the results of measuring the IL-6 level in the colon when the three types of lactic acid bacteria preparations of the present invention or the V company lactic acid bacteria preparations are administered to an animal model of colitis.

### MODE FOR CARRYING OUT INVENTION

Hereinafter, the present invention will be described in detail.

However, the following examples are only illustrative of the present invention, and the content of the present invention is not limited to the following examples.

### Example 1: Discovering a new combination of strain mixture

For various lactic acid bacteria strains isolated from infant feces, kimchi, and cheese, the individual efficacy of each strain was screened in various aspects. In addition, as a result of performing efficacy evaluation in various aspects using various strains in various combinations, the present inventors confirmed an unexpected synergistic effect with respect to the combination of three strains of *Lactobacillus johnsonii* IDCC9203 (Isolated from infant feces / Accession number KCTC 10923BP), *Lactobacillus plantarum* IDCC3501 (Isolated from kimchi / Accession number KCTC 13586BP) and *Bifidobacterium animalis* subspecies *lactis* IDCC4301 (Isolated from infant feces / Accession number KCTC 13587BP) in anti-inflammatory efficacy, as well as confirmed that the actual therapeutic efficacy is remarkable in terms of pharmacology for inflammatory bowel disease. The data presented below demonstrate the unexpected discovery of the strain combination of the three lactic acid bacteria preparations of the present invention.

### Example 2: Confirmation of anti-inflammatory synergistic effect of three types of lactic acid bacteria preparations (mixture) of the present invention, and comparison of strain combination specificity thereof

### 2-1. Confirmation of specificity of the composition of the three lactic acid bacteria preparations (mixture) of the present invention, and comparison of anti-inflammatory effects

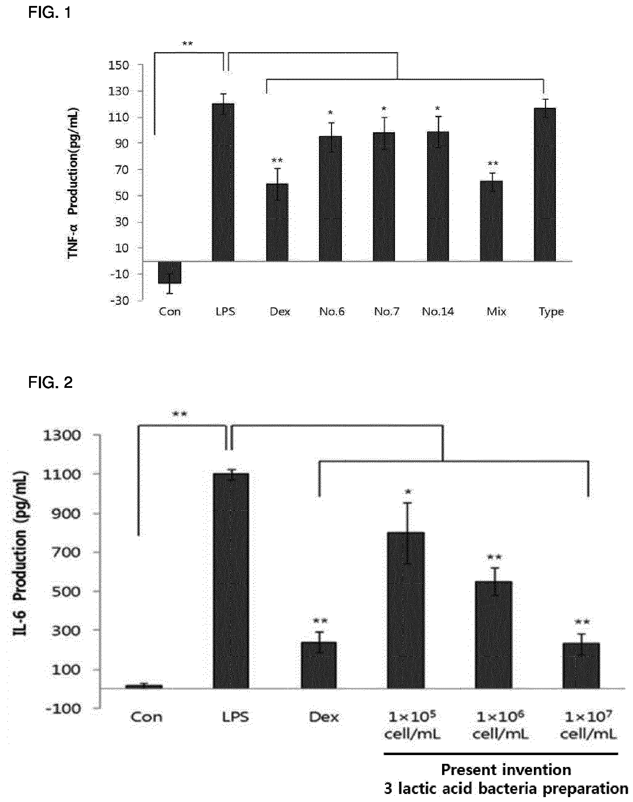

*Lactobacillus johnsonii* IDCC9203 (No. 6 in FIG. 1), *Lactobacillus plantarum* IDCC3501 (No. 7 in FIG. 1) and *Bifidobacterium animalis* subspecies *lactis* IDCC4301 (No. 14 in FIG. 1) each was cultured in MRS broth at 37°C for 2 hours, and then the cells were collected by centrifugation at 10,000×g for 15 minutes. After the cells were washed twice with 1X PBS buffer, the sample was killed by heating at 100°C for 10 minutes (heat-killed), and then used for anti-inflammatory effect analysis. The dead cell mixture for the three types of lactic acid bacteria was prepared by mixing each of the dead cell strains in a CFU standard of 1: 1: 1 ratio.

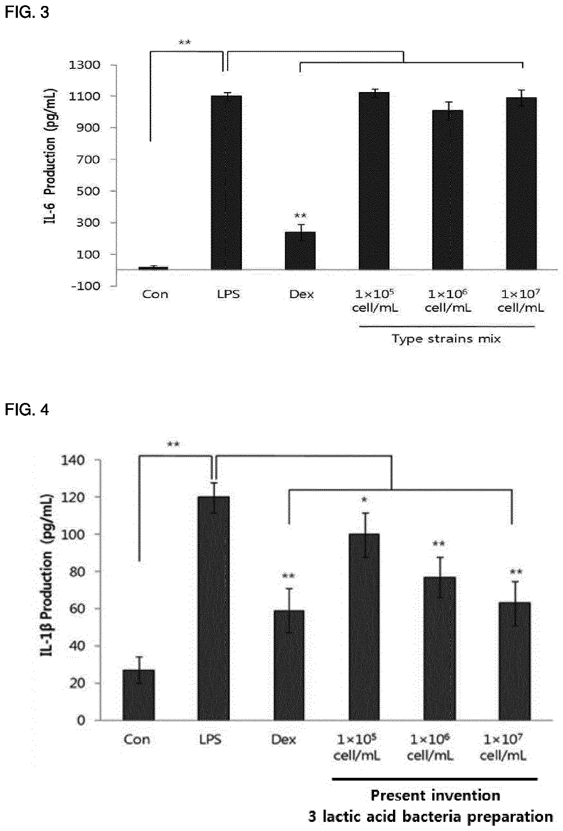

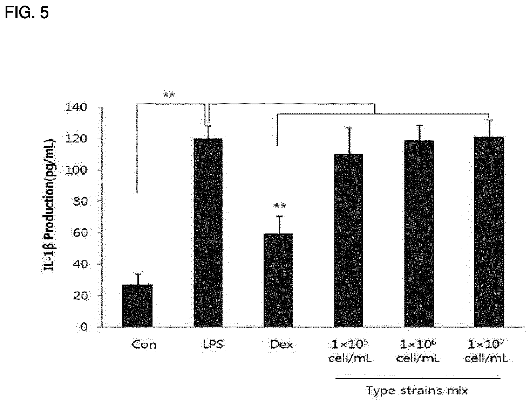

Various mixtures (i.e., mixtures of the same species but with different specific strains) were prepared in the same manner as in the present invention using other lactic acid bacteria of the same species as the constituent strains of the three lactic acid bacteria preparations (mixture) of the present invention, and efficacy was comparatively evaluated. As a representative example, FIG. 1, FIG. 3, and FIG. 5 shows the experimental results for the same type strain mixtures (*Lactobacillus johnsonii* KCTC 3801^{T}, *Lactobacillus plantarum* KCTC 3108^{T}, *Bifidobacterium animalis* ssp. *lactis* IDCC KCTC 3219^{T}) of the present invention.

RAW 264.7 cells, which are mouse macrophages, were cultured at 37°C and 5% CO₂ condition using phenol red-free Dulbecco's modified Eagle medium (DMEM, Gibco-BRL, Gaithersburg, MD, USA) containing 10% fetal bovine serum (FBS, Atlas, Fort Collins, CO, USA) and 1% penicillin (Sigma-Aldrich, St. Lousi, MO, USA).

In order to confirm the effect of the three lactic acid bacteria preparations (mixture) of the present invention on inflammatory cytokine production, the cultured Raw 264.7 cells were recovered using a scraper or trypsinize, put into a 15 ml falcon tube, and centrifuged at 500 rpm for 5 minutes. After removing the supernatant, 1 ml of complete media was added and resuspended, and the number of cells per ml was counted. 2 ml of the diluted cell solution was added to each well of a 6-well plate and cultured overnight for 24 hours. After sucktion of the medium in each well in a 6-well plate, it was washed with PBS. After dispensing 2 ml of a new medium, 4 ul of LPS (final LPS concentration is 2 ul/ml) was treated in all wells and in the first well, 1 mM dexamethasone was treated with 20 ul (final concentration of 10 uM/ml) as a positive control. PBS was treated as a negative control, the remaining wells were treated with LPS, and the three lactic acid bacteria preparations of the present invention were treated at a concentration of 1×10⁵ to 1×10⁷ cell/mL, followed by incubation overnight for 24 hours. The culture solution of each well was transferred to a 15 ml falcon tube and centrifuged at 9,500 rpm for 5 minutes. The supernatant of each tube was separately recovered and used for cytokine experiments. As an inflammatory cytokine, it was confirmed by using an ELISA kit (R&D system) whether or not to inhibit the production of TNF-α, IL-6, and IL-1β.

As a result of the experiment, it was confirmed that when the three types of lactic acid bacteria preparations (mixture) of the present invention were treated, the inflammatory cytokine production inhibitory effect (i.e. anti-inflammatory effect) was significantly increased compared to when the individual strains constituting the same were used alone. As a representative example showing this, as shown in FIG. 1, the TNF-α level by the treatment of the three lactic acid bacteria preparations (indicated as Mix in FIG. 1) of the present invention exhibited an effect of reducing the level to the same level as that of the dexamethasone treatment group, and it was confirmed that the reduction effect was significantly increased compared to when individual strains were treated alone.

In addition, as representatively shown in FIGs. 1, 3 and 5, when using a mixture using other strains of the same species, the TNF-α, IL-6, IL-1β production inhibitory effect was significantly less compared to that in the case of using the three types of lactic acid bacteria preparation (mixture) of the present invention, the dose-dependent anti-inflammatory effect was remarkably shown, in particular, in the 1×10⁷ cell/mL administration group, the anti-inflammatory effect was found to be equivalent to or similar to that of dexamethasone (See FIGs. 1, 2 and 4).

### 2-2. Comparison of NO production inhibitory ability of constituent strains

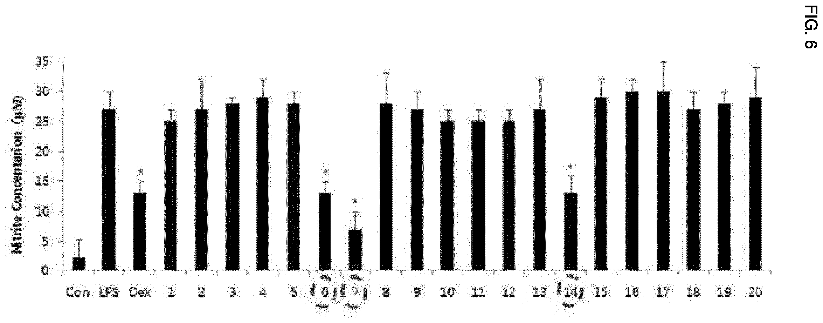

As a result of comparing the individual efficacy of each strain in various aspects for various lactic acid bacteria strains isolated from infant feces, kimchi and cheese, representatively, FIG. 6 shows the results of experiments comparing the efficacy of individual strains for the inhibition of NO (nitric oxide) production, and it shows comparatively experimental results for several representative strains, including *Lactobacillus johnsonii* IDCC9203 (No. 6 in FIGs. 1 and 6), *Lactobacillus plantarum* IDCC3501 (No. 7 in FIGs. 1 and 6) and *Bifidobacterium animalis* subspecies *lactis* IDCC4301 (No. 14 in FIGs. 1 and 6) used in the present invention.

The experimental method is simply as follows. Each of the lactic acid bacteria was cultured by the above-mentioned general culture method, and after completion of the culture, the cells were obtained by centrifugation, and the cells were killed by heat-killed at 100°C for 15 minutes. RAW 264.7 cells (1×10⁵/mL) were first cultured in DMEM, treated with LPS (2 µl/ml) and each heat-killed cells (1×10 cell⁵/mL), and then incubated for 24 hours. The concentration of NO was investigated by measuring the amount of nitrite in the cell culture supernatant using Griess reagent (Sigma, USA) according to the manufacturer's protocol. After mixing 150 µL of cell culture supernatant obtained from RAW264.7 cell medium treated as described above and incubated for 24 hours and 150 µL of Griess reagent, it was centrifuged at 1,000xg for 10 minutes, and then incubated for 10 minutes at room temperature. Absorbance was measured at 595 nm using a microplate reader, and was compared based on a calibration curve formed through sodium nitrite.

*Lactobacillus johnsonii* IDCC9203, (No. 6 in FIGs. 1 and 6), *Lactobacillus plantarum* IDCC3501 (No. 7 in FIGs. 1 and 6) and *Bifidobacterium animalis* subspecies *lactis* IDCC4301 (No. 14 in FIGs. 1 and 6), a constituent of the present invention, was significantly superior in NO production inhibition ability compared to other lactic acid bacteria strains (see FIG. 6).

### Example 3: Confirmation of in vivo inflammatory bowel disease (IBD) treatment efficacy of three kinds of probiotic preparations of lactic acid bacteria of the present invention & comparison of efficacy with sulfasalazine, a compound for treating IBD

### Experimental Method

### 1) Preparation of three kinds of probiotic preparations of lactic acid bacteria of the present invention

The three lactic acid bacteria probiotic preparations of the present invention used in the experiment were prepared by mixing three live strains of *Lactobacillus johnsonii* IDCC9203 (KCTC 10923BP), *Lactobacillus plantarum* IDCC3501 (KCTC 13586BP) and *Bifidobacterium animalis* subspecies *lactis* IDCC4301 (KCTC 13587BP) in a CFU (colony-forming unit) standard 1: 1: 1 ratio. Each live strain contained 2×10¹² CFU/g. Each bacteria was cultured in a growth medium at 37°C for 16 hours until the exponential phase, and then the bacterial pellet was stored in a freeze dryer and freeze-dried (Condition at -80°C under vacuum, overnight). The freeze-dried three kinds of lactic acid bacteria preparations of the present invention were stored at 4°C until use.

### 2) Experimental animals

Female BALB/c mice (8 weeks old) were purchased from Orient Bio (Seongnam, Republic of Korea). Five animals per cage were housed in an environmentally controlled facility (temperature 22°C±2°C, humidity 55%±5%) with a 12/12 h light/dark cycle.

Animal care and treatment was carried out in accordance with the guidelines established by the National Institutes of Health Animal Research and Care, and it was approved by the Animal Experiment Ethics Committee (Institutional Animal Care and Use Committee, Approval No.: A1611-2) of Ildong Pharmaceutical Co., Ltd.

### 3) Mouse colitis model

A mouse model of colitis induced by DSS (molecular weight 36-50 kDa/MP biochemicals, Santa Ana, CA, USA) was used in this study. After 7 days of adaptation period, as shown in Table 1, mice were divided into 7 groups (10 mice per group), and the average of the initial body weight (BW) was similarly adjusted. DSS was dissolved in drinking water at a concentration of 3.5% (w/v), and all mice except the normal group were supplied from day-0 to day-8. Animals received water and food ad libitum.

200 µl of distilled water (DW) was orally administered to vehicle group mice. The three lactic acid bacteria preparations of the present invention have a dose range of 10⁶ CFU/mice/day - 10⁹ CFU/mice/day, and sulfasalazine (Tokyo chemical industry, Tokyo, Japan) as a positive control is at a concentration of 500 mg/kg (BW)/day, after suspending in DW, it was administered in the same volume and in the same manner as the vehicle group. Table 1 below summarizes the material treatment conditions for each experimental group and control group.

**Table 1**

| *Group* | *Drinking water* | *Administration* |
|---|---|---|
| Normal | D W | DW |
| Vehicle | 3.5% DSS | DW |
| ID-JPL 10⁶ | 3.5% DSS | ID-JPL 10⁶ CFU per mouse per day |
| ID-JPL 10⁷ | 3.5% DSS | ID-JPL 10⁷ CFU per mouse per day |
| ID-JPL 10⁸ | 3.5% DSS | ID-JPL 10⁸ CFU per mouse per day |
| ID-JPL 10⁹ | 3.5% DSS | ID-JPL 10⁹ CFU per mouse per day |
| Sulfasalazine | 3.5% DSS | Sulfasalazine 500 mg per kg BW per day |

| | | |
|---|---|---|
| BW, body weight; CFU, colony-forming units; DSS. dextran sulfate sodium; DW, distilled water. | | |

BW (weight) and stool status were observed once a day for each mouse, and a DAI (disease activity index) score was calculated according to the criteria described in Table 2 below as in a previously known method. The maximum value that could be calculated according to Table 2 below was 12. After euthanasia with carbon dioxide gas, the large intestine (colon) from the ileocecal junction to the anus was removed and the length was measured, and was washed with phosphate buffered saline (PBS) in order to remove the intestinal residue. One third of the distal colon was fixed with 10% formaldehyde solution and used for histopathological analysis. The remaining two-thirds of the distal colon tissue was used for protein extraction to measure proinflammatory cytokine levels and stored at -80°C until use.

**Table 2**

| *Score* | *Weight loss (%)* | *Stool consistency* | *Visible blood in feces* |
|---|---|---|---|
| 0 | None | Normal | None |
| 1 | 1-5 | | |
| 2 | 6-10 | Pasty stools | Slight bleeding |
| 3 | 11-20 | | |
| 4 | >20 | Diarrhea | Gross bleeding |

### 4) Histological analysis

Colonic tissue was fixed with a 10% neutral buffered formaldehyde solution, praffin embedding was carried out in a conventional manner. Paraffin-embedded tissue sections were cut to a thickness of 4 µm and stained with hematoxylin & eosin (H & E). H&E-stained colon tissues were observed at 100x and 200x magnifications using a Nikon ECLIPSE 50i optical microscope (Nikon, Tokyo, Japan), and as described in Table 3 (Histological Scoring System), histological evaluation was performed by two pathologists according to commonly known parameters. According to Table 3, the maximum score calculated by summing each of the three parameters was 10.

**Table 3**

| *Feature* | *Score* | *Description* |
|---|---|---|
| Severity of inflammation | 0 | None |
| | 1 | Mild |
| | 2 | Moderate |
| | 3 | Severe |
| Extent of inflammation | 0 | None |
| | 1 | Mucosa |
| | 2 | Mucosa and submucosa |
| | 3 | Transmural |
| Crypt damage | 0 | None |
| | 1 | 1/3 damaged |
| | 2 | 1/2 damaged |
| | 3 | Crypts lost, surface epithelium present |
| | 4 | Crypts and surface epithelium lost |

### 5) Measurement of colonic pro-inflammatory cytokine levels

Colonic proteins were extracted using RIPA buffer (Upstate, Temesula, CA, USA) added with a protease inhibitor (Sigma-Aldrich, St. Louis, MO, USA). After adding 300 µl of RIPA buffer to colon tissue, the tissue was homogenized on ice for 1 minute, and centrifuge at 20,000g for 15 min at 4°C. After collecting the supernatant, quantification was performed using a bicinchoninic acid protein assay reagent kit (Pierce, Rockford, IL, USA). An ELISA kit (R&D Systems, Minneapolis, MN, USA) was used according to the manufacturer's protocol to detect the levels of proinflammatory cytokines such as TNF-a, IL-1b and IL-6 in colon tissues. Each cytokine value was corrected for the total amount of each colon protein sample.

### 6) Statistical analysis

Results are presented as mean ± standard error, and each result was processed using a statistical analysis system, Prism 7 (GraphPad Software, San Diego, CA, USA). All results represent the average of at least three independent experiments. Statistical comparisons between the vehicle group and each treatment group were performed by one-way analysis of variance and subsequent Dunnett's test. P value < 0.05 was considered statistically significant.

### Experimental Results

### 3-1. Effects of 3 types of lactic acid bacteria preparations of the present invention on colitis symptoms in vivo

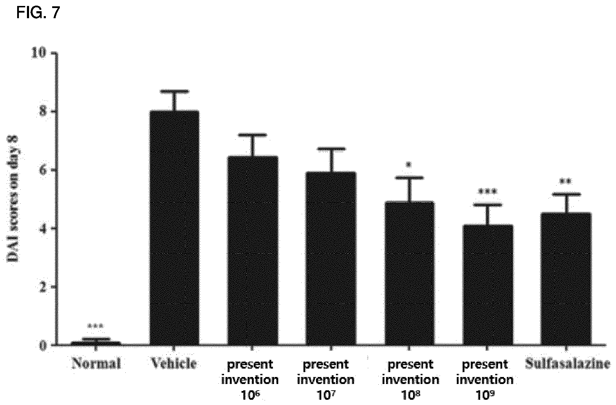

To determine the severity of colitis, the BW and defecation status of each mouse were observed once a day and the DAI score was recorded. As the amount of DSS provided to the mice increased, the incidence of bloody stool and diarrhea increased, whereas body weight (BW) gradually decreased. In the vehicle group, from the 4th day of the experiment, the DAI score was shown to rise steadily. Administration of the three lactic acid bacteria preparations of the present invention dose-dependently decreased the DAI score (see FIG. 7), and showed effects of preventing weight loss, improving the appearance of excreta, and improving bloody stool. The DAI score of the group administered with the three lactic acid bacteria preparations of the present invention at a dose of 10⁸ CFU/mice/day or 10⁹ CFU/mice/day was similar to the DAI score of the sulfasalazine administration group.

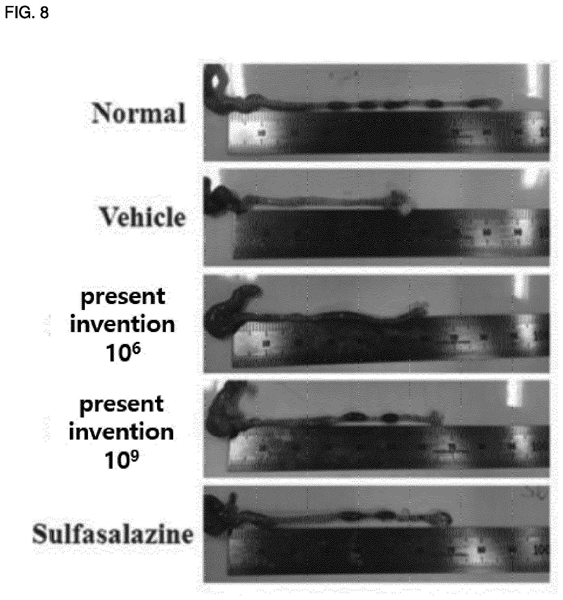

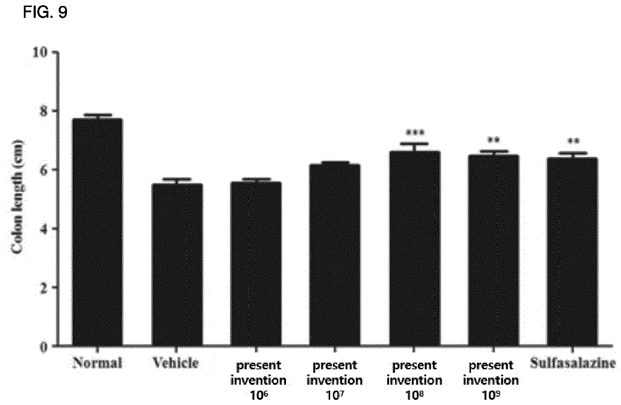

In mice induced with colitis, the length of the colon was shorter than in the normal group. This symptom of shortening of the colon length was prevented in a dose-dependent manner by the three types of lactic acid bacteria preparations of the present invention, and it showed an equivalent effect pattern between 10⁸ CFU/mice/day or 10⁹ CFU/mice/day dose administration group of the three lactic acid bacteria preparations of the present invention and the sulfasalazine administration group (see FIGs. 8 and 9).

### 3-2. Effect of 3 types of lactic acid bacteria preparations of the present invention on colon histological parameters

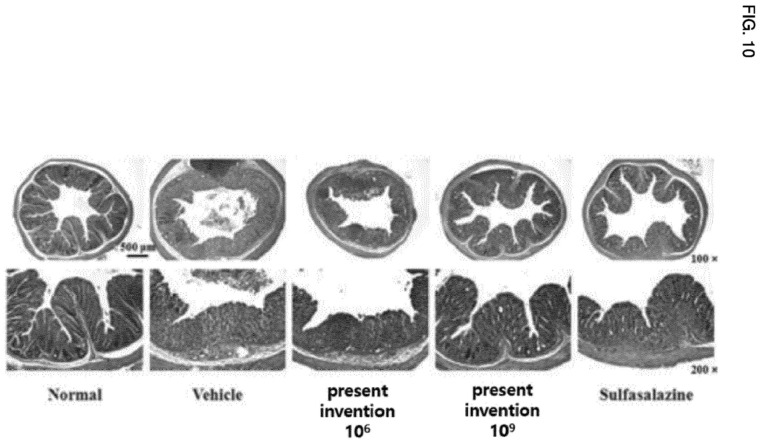

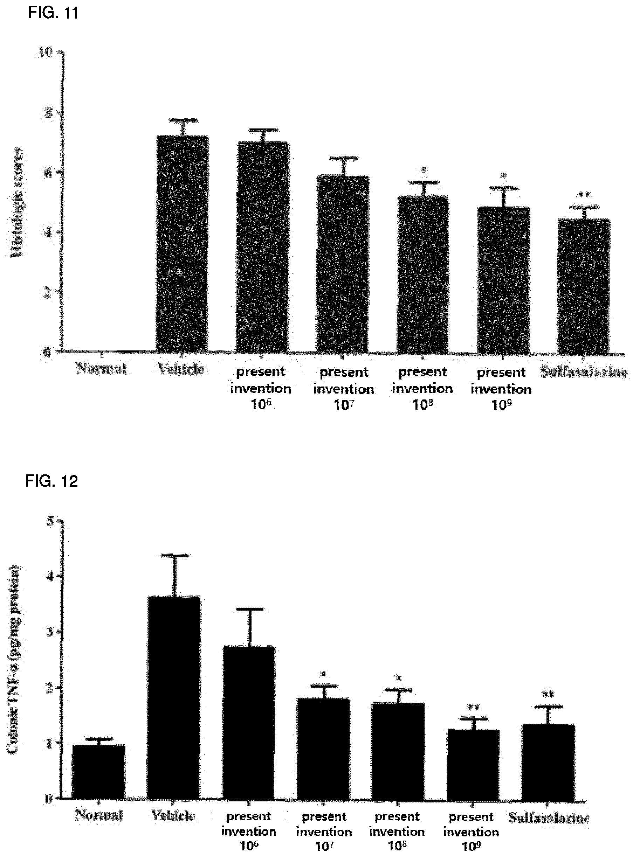

To evaluate the severity of histological damage of DSS-induced colitis, H&E-stained colon tissues were observed using a microscope, and histologic scores were measured. As shown in FIGs. 10 and 11, infiltration of inflammatory cells in the mucosa and submucosal tissue, severe intestinal crypt damage, and loss of goblet cells and epithelial cells were observed in the vehicle group. Also, histological score increased to 7.2±0.6. On the other hand, the histological score in the sulfasalazine-administered group decreased sharply. In the group treated with the three lactic acid bacteria preparations of the present invention, the histological score was decreased in a dose-dependent manner. In particular, the histological score of colon damage was significantly reduced in the 10⁸ CFU/mice/day administration group and the 10⁹ CFU/mice/day administration group of the three lactic acid bacteria preparations of the present invention (5.2±0.5 and 4.9±0.6, respectively), and this was similar to that in the sulfasalazine administration group (4.5±0.5).

### 3-3. Effects of the three lactic acid bacteria preparations of the present invention on the expression of proinflammatory cytokines in the colon

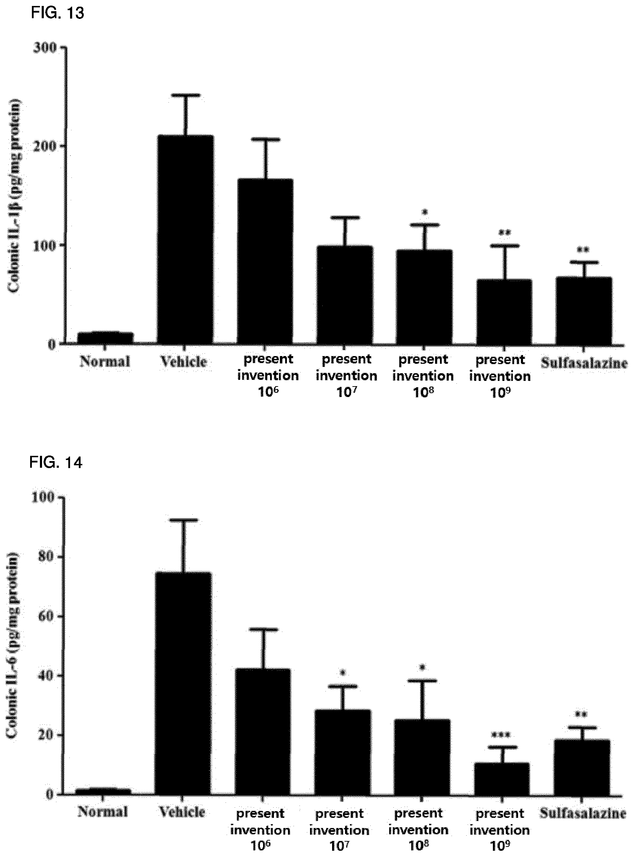

To detect the levels of colonic cytokines associated with the inflammatory response, ELISA was performed. Representatively, the expression patterns of three pro-inflammatory cytokines, TNF-α, IL-1β, and IL-6, were investigated, and they are known to play an important role in the pathology of colitis. As shown in FIGs. 12, 13 and 14, although the expression level of each of the cytokines was increased in the colitis pathology, It was reduced in a dose-dependent manner by administration of the three lactic acid bacteria preparations of the present invention. Even compared with the sulfasalazine administration group used as a positive control, the 10⁹ CFU/mice/day administration group of the three lactic acid bacteria preparations of the present invention showed a similar inhibitory effect.

### Example 4: Comparative evaluation of the inflammatory bowel disease treatment efficacy of the existing commercial probiotic products approved for IBD and the three types of lactic acid bacteria preparations of the present invention

### Experimental Method

The effect on the improvement of inflammatory bowel disease (IBD) was compared with the three probiotic preparations (mixture) of the present invention using company V's lactic acid bacteria preparations, which is the first and only probiotic product in Korea that has been individually recognized for 'helping intestinal health by regulating intestinal immunity' by the Ministry of Food and Drug Safety, currently. The lactic acid bacteria preparation of V Company is known to consist of a total of about 450 billion live bacteria in one pouch (4.5 × 1011 bacteria/pack), and specifically, it is known that 8 types of live bacteria strains of *Lactobacillus paracasei* DSM 24734, *Lactobacillus plantarum* DSM 24730, *Lactobacillus acidophilus* DSM 24735, *Lactobacillus delbrueckii* subsp. *bulgaricus* DSM 24734, *Bipidobacterium longum* DSM 24736, *Bipidobacterium breve* DSM 24732, *Bipidobacterium infantis* DSM 24737, *Streptococcus salivarius* subsp. *thermophilus* DSM 24731 are mixed.

The test group composition was performed as shown in Table 4 below, at this time, the three strains described above as a live cell mixture of three types of lactic acid bacteria of the present invention in a ratio of 1:1:1 based on CFU was typically used for the experiment. Except for the conditions described in Table 4 below, the rest of the experimental method was performed in the same manner as in Example 3.

**Table 4**

| Group | Number of animals | Drinking water | Route of administration | Dose amount | Dosage |
|---|---|---|---|---|---|
| Normal Control | 10 | Primary Distilled water | p.o | 10 mL/kg/day | 0 mg/kg/day |
| Vehicle | 10 | Primary distilled water with 3.5% DSS added | p.o | 10 mL/kg/day | 0 mg/kg/day |
| V Company lactic acid bacteria preparation | 10 | | p.o | 200ul/mouse/ day | 5.4×10⁸CFU/ day |
| Present invention 10⁸ | 10 | | p.o | 200ul/mouse/ day | 10⁸CFU/day |
| Present invention 10⁹ | 10 | | p.o | 200ul/mouse/ day | 10⁹CFU/day |

### Experimental Results

### 4-1. Comparison of effects on colitis symptoms in vivo

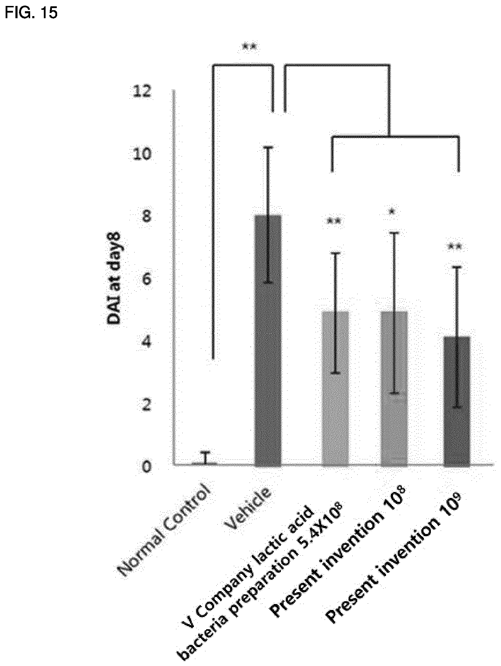

As shown in FIG. 15, the experimental results, the effect of preventing weight loss, improving the appearance of excretion and improving bloody stool was shown in the group administered with the three lactic acid bacteria preparations of the present invention and the V-company lactic acid bacteria preparations, accordingly, the DAI score was decreased. In particular, when comparing the 10⁸ CFU administration group of the three types of lactic acid bacteria preparations of the present invention, despite being administered at a lower CFU dose compared to the V company lactic acid bacteria preparations 5.4×10⁸ CFU administration group, the DAI socre was reduced to the same level as compared to the V company lactic acid bacteria preparation.

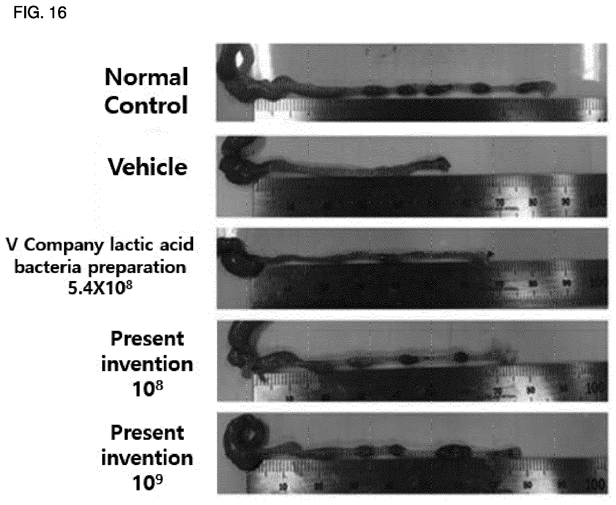

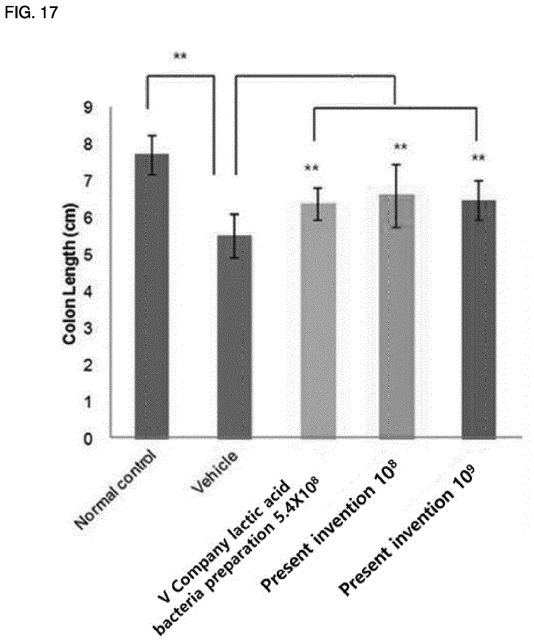

In addition, as shown in FIGs. 16 and 17, it was confirmed that the three types of lactic acid bacteria preparations of the present invention have a remarkable effect of preventing or improving colon length shortening caused by colitis.

### 4-2. Comparison of colon histological parameters

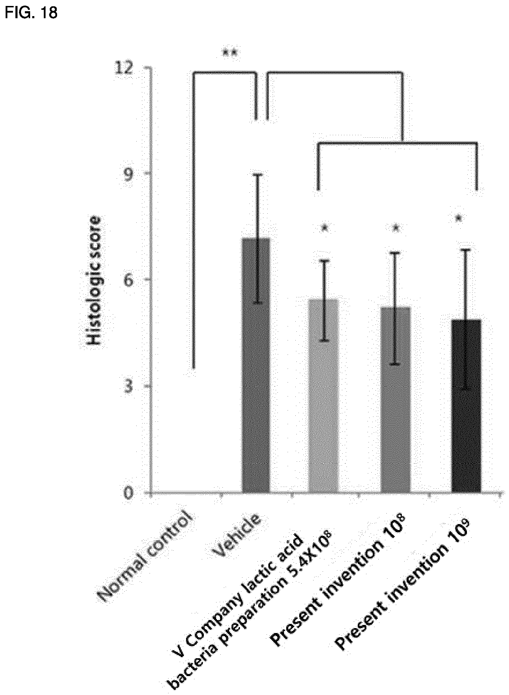

As representatively shown in FIG. 18, in the vehicle group, infiltration of inflammatory cells in mucosal and submucosal tissues, severe intestinal crypt damage, and loss of goblet cells and epithelial cells were observed, whereas in the group treated with the three types of lactic acid bacteria preparations of the present invention and the group administered with the V company lactic acid bacteria preparations, the histological score of colon damage was reduced, showing the effect of restoring colon tissue. In particular, when comparing the three types of lactic acid bacteria preparation 10⁸ CFU administration group of the present invention, in spite of being administered at a lower CFU dose compared to the V company lactic acid bacteria preparations 5.4×10⁸ CFU administration group, it showed the prevention or / and treatment effect of colon damage at the same level compared with the lactic acid bacteria of the V company.

### 4-3. Comparison of effects on the expression of proinflammatory cytokines in the colon

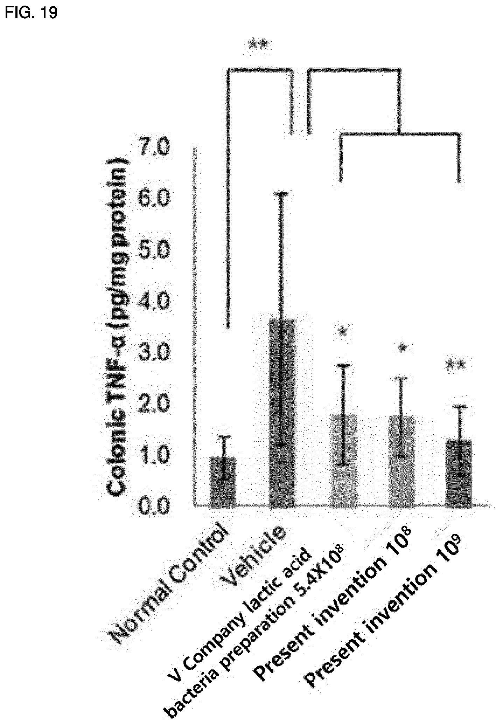

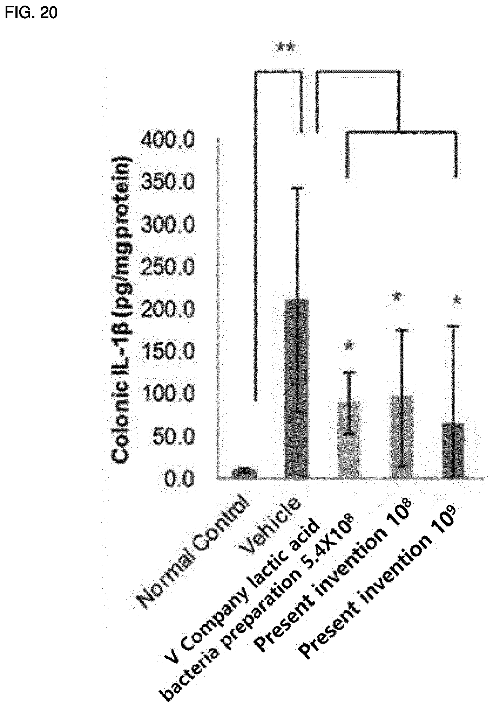

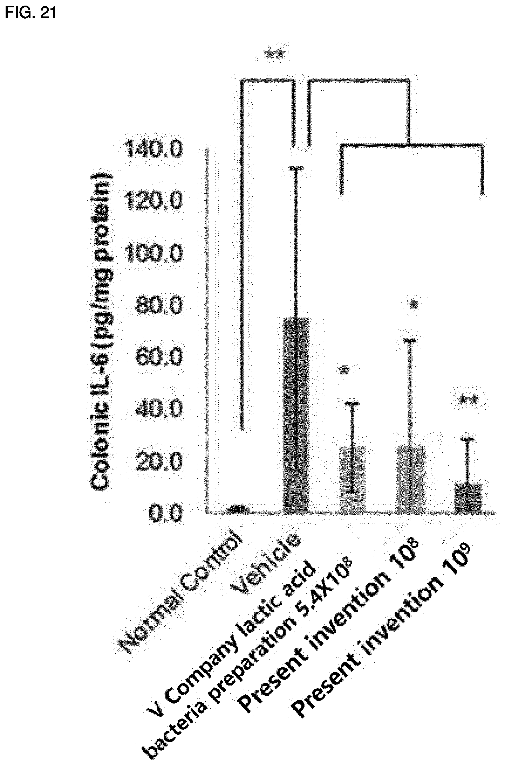

Representatively, as shown in FIGs. 19, 20 and 21, ELISA was performed to detect the level of colonic cytokines related to the inflammatory response, and it was confirmed that the increased expression of proinflammatory cytokines of TNF-α, IL-1β and IL-6 in the colitis pathology was significantly lowered in the group treated with the three lactic acid bacteria preparations of the present invention and the group administered with the V company lactic acid bacteria preparations. In particular, when comparing the three types of lactic acid bacteria preparation 10⁸ CFU administration group of the present invention, in spite of being administered at a lower CFU dose compared to the V company lactic acid bacteria preparations 5.4×10⁸ CFU administration group, it showed the prevention or / and treatment effect of colon damage at the same level compared with the lactic acid bacteria of the V company.

### INDUSTRIAL APPLICABILITY

As described above, the present invention relates to a novel probiotic composition for regulating intestinal immunity, and more particularly, it relates to a composition for inhibiting inflammation and/or preventing, improving, or treating inflammatory bowel disease, comprising *Lactobacillus johnsonii, Lactobacillus plantarum,* and *Bifidobacterium animalis* subspecies *lactis* as an active ingredient.

The composition comprising the unique combination of three types of lactic acid bacteria of the present invention has remarkable preventive and therapeutic effects for inflammatory bowel disease (IBD), and in particular, these effects are pharmacologically superior compared to known IBD therapeutic drugs (e.g., sulfasalazine). In addition, since the composition of the present invention is a probiotic composition, it has a great advantage as a safe composition with very little risk of side effects, and thus has high industrial applicability.

### [Accession number]

Name of deposit institution: Korea Research Institute of Bioscience and Biotechnology
accession number: KCTC10923BP
deposit date : 2006.03.15

Name of deposit institution: Korea Research Institute of Bioscience and Biotechnology
accession number: KCTC13586BP
deposit date : 2018.07.17

Name of deposit institution: Korea Research Institute of Bioscience and Biotechnology
accession number: KCTC13587BP
deposit date : 2018.07.17

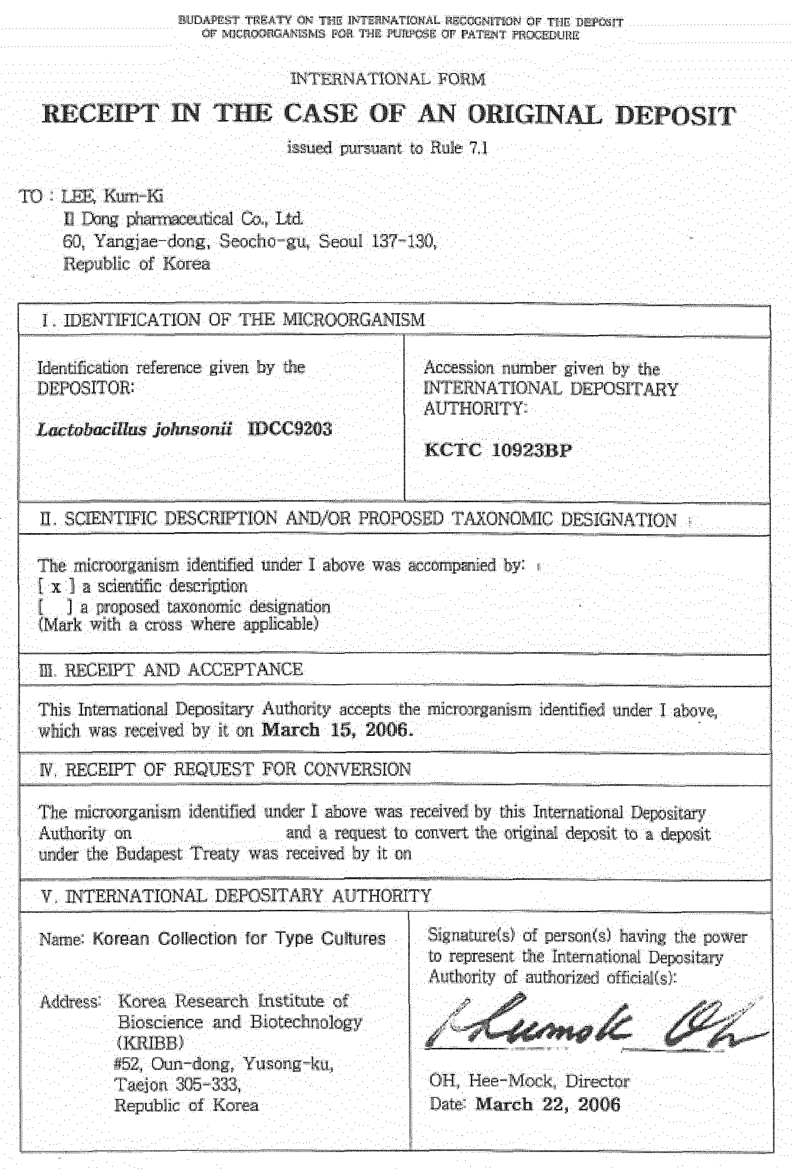

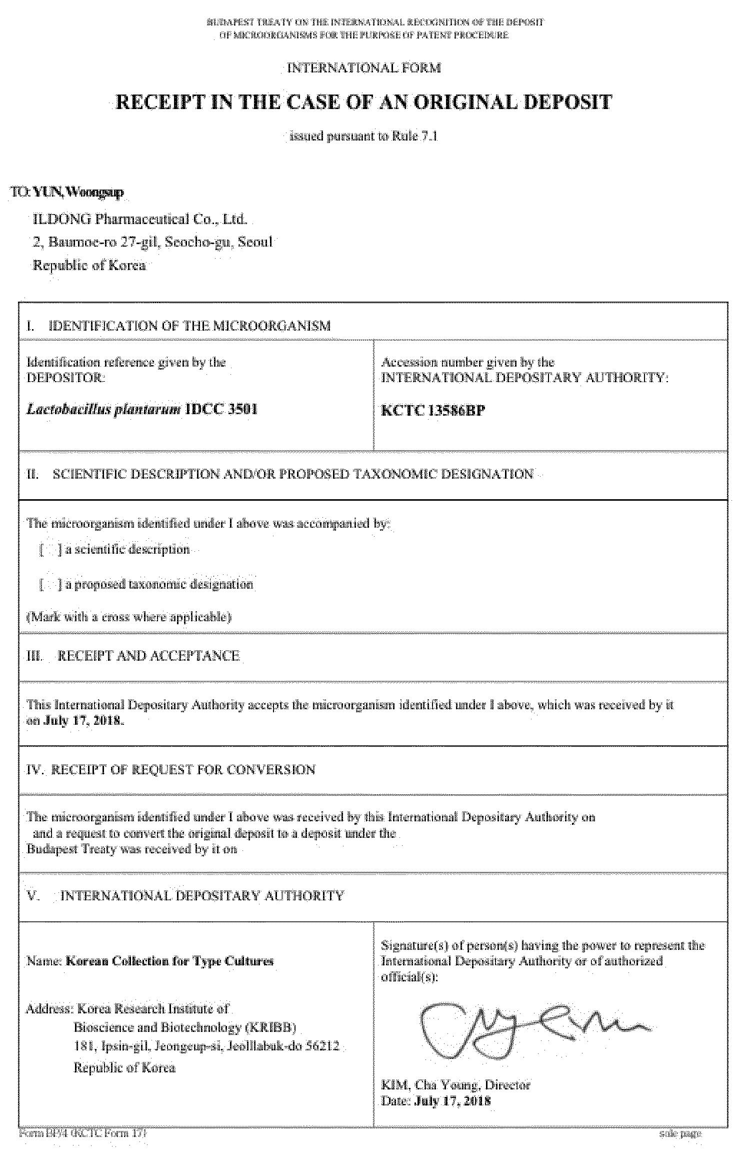

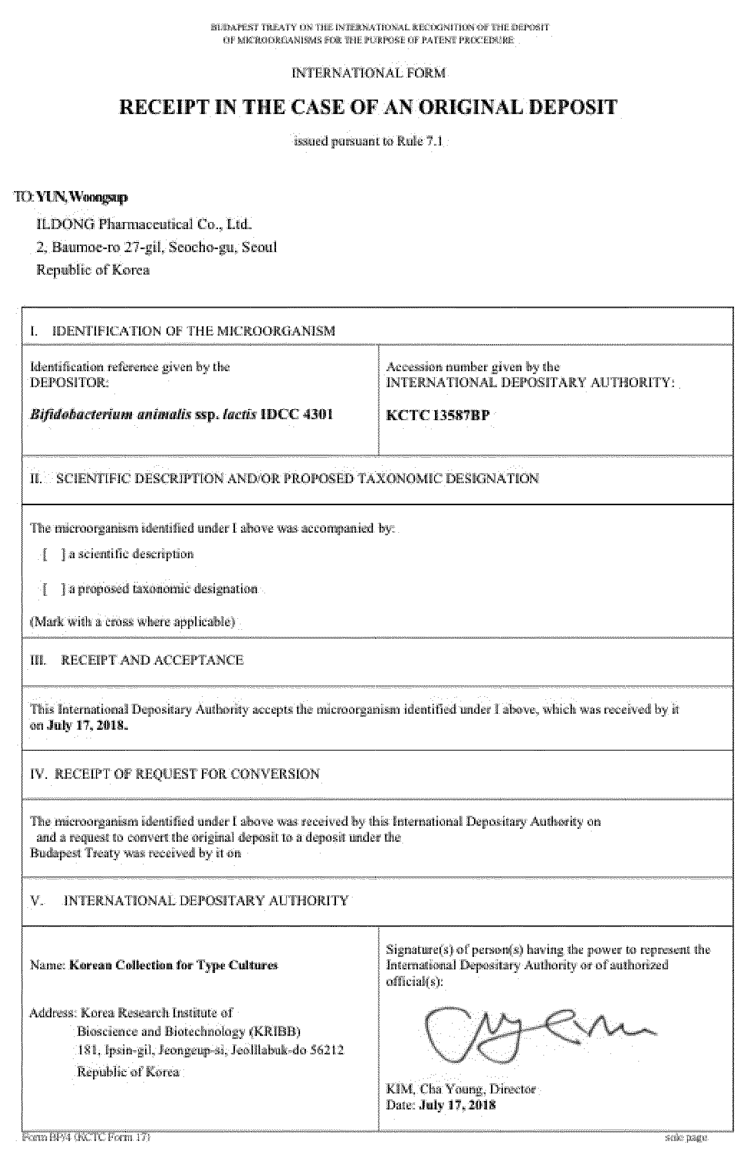

## Claims

1. A pharmaceutical composition for preventing or treating inflammatory bowel disease, comprising *Lactobacillus johnsonii, Lactobacillus plantarum* and *Bifidobacterium animalis* subspecies *lactis* as an active ingredient.

2. The composition according to claim 1, wherein the composition comprises a *Lactobacillus johnsoni*i IDCC9203 strain, a *Lactobacillus plantarum* IDCC3501 strain, and a *Bifidobacterium animalis* subspecies *lactis* IDCC4301 strain.

3. The composition according to claim 1, wherein the *Lactobacillus johnsonii, Lactobacillus plantarum* and *Bifidobacterium animalis* subspecies *lactis* are live cells or dead cells, respectively.

4. A food composition for preventing or improving inflammatory bowel disease, comprising *Lactobacillus johnsonii, Lactobacillus plantarum,* and *Bifidobacterium animalis* subspecies *lactis* as an active ingredient.

5. The composition according to claim 4, wherein the *Lactobacillus johnsonii, Lactobacillus plantarum,* and *Bifidobacterium animalis* subspecies *lactis* are live cells or dead cells, respectively.

6. A pharmaceutical composition for anti-inflammatory, comprising *Lactobacillus johnsonii, Lactobacillus plantarum,* and *Bifidobacterium animalis* subspecies *lactis* as an active ingredient.

7. A food composition for anti-inflammatory, comprising *Lactobacillus johnsonii, Lactobacillus plantarum,* and *Bifidobacterium animalis* subspecies *lactis* as an active ingredient.

8. Use of a composition comprising *Lactobacillus johnsonii, Lactobacillus plantarum* and *Bifidobacterium animalis* subspecies *lactis* as an active ingredient for preparing a formulation for the prevention or treatment of inflammatory bowel disease.

9. A method of treating inflammatory bowel disease, comprising administering to a subject in need thereof an effective amount of a composition comprising *Lactobacillus johnsonii, Lactobacillus plantarum* and *Bifidobacterium animalis* subspecies *lactis* as an active ingredient.

10. The method according to claim 9, wherein the inflammatory bowel disease is one or more selected from the group consisting of Crohn's disease, ulcerative colitis, intestinal Behcet's disease, and ischemic enteritis.

11. Use of a composition comprising *Lactobacillus johnsonii, Lactobacillus plantarum* and *Bifidobacterium animalis* subspecies *lactis* as an active ingredient for preparing a formulation for preparing an anti-inflammatory agent.

12. A method of inhibiting inflammation comprising administering to a subject in need thereof an effective amount of a composition comprising *Lactobacillus johnsonii, Lactobacillus plantarum* and *Bifidobacterium animalis* subspecies *lactis* as an active ingredient.
